# EUROPÄISCHE PATENTANMELDUNG

(11) **EP 4 706 569 A1**
(43) Veröffentlichungstag der Anmeldung: **11.03.2026**
(21) Anmeldenummer: 24198682.7
(22) Anmeldetag: 05.09.2024
(51) Int. Cl.: A61B 18/12, G01R 19/10, A61B 18/14, A61B 18/00

(54) **ELEKTROCHIRURGISCHES GERÄT MIT STROMSENSOR FÜR BEHANDLUNGSSTROM**

(71) Anmelder: Erbe Elektromedizin GmbH, 72072 Tübingen (DE)
(72) Erfinder: WERNER, Erich, 72827 Wannweil (DE); PUGGÉ, Mike, 72658 Bempflingen (DE)
(74) Vertreter: Rüger Abel Patentanwälte PartGmbB

(57) **Zusammenfassung**

Erfindungsgemäß wird für einen Stromsensor (23) eines Geräts (10) zur Speisung eines Instruments (11, 11') ein ringförmiger Magnetkreis (25) vorgesehen, durch den zwei das Instrument (11) und eine Neutralelektrode (15) oder das Instrument (11') speisende Leitungen (19, 21) geführt sind. Die Mäntel (28, 30) der beiden Leitungen (19, 21) weisen eine in ihrer Durchschlagfestigkeit auf die Spannungsbelastung der Leitungen (19, 21) bemessene hinreichende Dicke auf. Diese ist jedoch so weit begrenzt, dass zwischen die beiden Leitungen (19, 21) noch ein Isolierkörper (31) eingesetzt werden kann, der wie die Mäntel (28, 30) aus einem unpolaren hochisolationsfähigen Isolatormaterial besteht, beispielsweise einem Kunststoff mit geringem Verlustwinkel. Der Isolierkörper (31, 31a) bis (31d), reduziert Teilentladungen im Bereich des Stromsensors (23), die ansonsten zu elektromagnetischen Emissionen und somit zur Störung des von dem Stromsensor (23) erzeugten Signals (24) führen könnten.

## Beschreibung

Die Erfindung betrifft ein elektrochirurgisches Gerät mit einem Generator zur Speisung eines elektrochirurgischen Instruments, mit dem Eingriffe an einem menschlichen oder tierischen Patienten vornehmbar sind.

Zur Behandlung des menschlichen oder tierischen Körpers sind monopolare oder bipolare Instrumente bekannt, die unter erheblicher Spannung mit hochfrequentem Strom (HF-Strom) gespeist werden. Die Spannung kann von mehreren 10 V bis zu mehreren 1000 V gehen. Dabei ist es häufig erforderlich, den zu dem Instrument gelieferten Strom präzise und auch mit hoher zeitlicher Auflösung zu messen, um den Generator entsprechend steuern zu können.

Aus der Praxis ist dazu ein Stromsensor bekannt, der einen Magnetkreis aufweist, durch den sowohl die zu dem Instrument führende wie auch von dem Instrument oder Patienten zurückführende Leitung geführt sind. Eine ebenfalls auf dem Magnetkreis vorgesehene Sensorspule oder auch andere Mittel zur Erfassung eines Magnetfelds liefern ein Signal, das den erfassten Strom kennzeichnet.

Zwischen beiden Leitungen kann je nach Behandlungsart eine erhebliche Spannung vorhanden sein, die insbesondere bei ungünstigen Einsatzbedingungen, beispielsweise Einsatzbedingungen in großer Höhe, bei niedrigem Luftdruck, hoher Luftfeuchtigkeit, usw., zu Teilentladungen innerhalb der Isolation der in enger Nachbarschaft zueinander geführten Leitungen führen kann. Diese Teilentladungen zwischen den beiden hochfrequenzstromführenden Leitungen können Störungen verursachen, die zu verfälschten Messungen und sogar Fehlermeldungen der angeschlossenen Sensorik führen. Dies auch dann, wenn sie so gering sind, dass sie im Hinblick auf die Durchschlagfestigkeit der Isolierung unbedenklich sind.

Einer Vergrößerung der Isolationsdicke der einzelnen Leitungen stehen die beengten Platzverhältnisse in entsprechenden Stromsensoren entgegen.

Davon ausgehend ist es Aufgabe der Erfindung, ein Gerät zur Speisung eines elektrochirurgischen Instruments anzugeben, das auch unter widrigen Einsatzbedingungen eine sichere Erfassung des zum Instrument gelieferten Stroms ermöglicht.

Das erfindungsgemäße Gerät umfasst einen Generator mit einem Auskoppelkreis, der über Leitungen mit den Geräteausgängen verbunden ist. Die Leitungen führen durch eine zentrale Öffnung eines Magnetkreises eines Stromsensors, wobei zwischen den beiden Leitungen ein Isolierkörper angeordnet ist. Dieser Isolierkörper maximiert die Distanz zwischen den beiden Leitungen, deren eigene Isolation dabei vorzugsweise auf ein vertretbares Minimum reduziert ist. Die Isolationsdicke der Leitungen und das Isolationsmaterial sind dabei vorzugsweise so gewählt, dass ausreichend Sicherheit gegen Spannungsdurchschlag gegeben ist.

Der zusätzliche zwischen den Leitungen in der zentralen Öffnung des Stromsensors angeordnete Isolierkörper vergrößert hierbei den Abstand der beiden Leitungen, insbesondere den Abstand deren beider Leiter, voneinander und reduziert somit sowohl den kapazitiven Leckstrom als auch etwaige Teilentladungen. Somit können elektromagnetische, von den Teilentladungen ausgehende Störungen reduziert und die Präzision und Sicherheit der Stromerfassung gesteigert werden. Insbesondere kann die zeitliche Auflösung des Stromsensors erhöht werden, weil kurzfristige Fluktuationen der Messwerte, d.h. das Messwertrauschen verringert werden. Der Stromsensor bleibt insbesondere auch bei widrigen Umweltbedingungen, wie reduzierter Luftdruck auf Höhen von 3000 m bis 5000 m über Normalnull und/oder erhöhter Luftfeuchtigkeit uneingeschränkt funktionsfähig.

Der Isolierkörper minimiert die Teilentladung im Bereich des Stromsensors beim Messen von HF-Strömen (250 kHz bis 2,5 MHz). Der zusätzliche Isolierkörper vergrößert die Distanz der beiden Leitungen bei nur begrenzt zur Verfügung stehenden Raum, insbesondere in der Öffnung des Stromsensors.

Als Isolationsmaterial für den Stromsensor wird vorzugsweise ein unpolares Isolationsmaterial wie PE, PP, PTFE oder PI verwendet, das bei hohen Frequenzen einen hohen spezifischen Durchgangswiderstand sowie minimale Wechselspannungsverluste aufweist. Somit erwärmt es sich nicht wesentlich und lässt kaum einen Stromfluss zu. Aufgrund dessen wird eine innere Teileentladung stark unterdrückt. Vorzugsweise ist der Isolierkörper dabei wenigstens etwas elastisch ausgebildet, um eine einfache Handhabung und eine örtliche Festlegung der beiden Leitungen in der Öffnung zu ermöglichen.

Sowohl für die Isolierung der Leitung als auch für den Isolierkörper wird vorzugsweise ein Material mit hoher Durchschlagsspannungsfestigkeit (zum Beispiel Silikon oder PTFE) verwendet. Damit wird auch die Spannungsfestigkeit der beiden Leitungen gegenüber benachbarten Potentialen außerhalb des Sensors sichergestellt.

Der in dem Stromsensor zwischen den beiden Leitungen in der Öffnung angeordnete Isolierkörper wirkt als Abstandshalter, der die beiden durch die Öffnung geführten Leitungen auf maximalen Abstand bringt. Prinzipiell kann der Isolierkörper aus anorganischem Material, wie beispielsweise Keramik bestehen, welches keine Teilentladung zulässt und die beiden Leitungen hinsichtlich der Teilentladung optimal trennt. Es können aber, wie oben erwähnt, auch unpolare Materialen zum Beispiel PTFE, Nylon usw., eingesetzt werden, die auf Wechselspannung ebenfalls nur schwach reagieren und bei denen aufgrund des geringen in der Isolation fließenden Stroms eine Teilentladung weitestgehend unterbunden wird.

Die beiden Leitungen weisen jeweils einen zentralen elektrischen Leiter in Gestalt eines einzelnen Drahts oder eines Drahtbündels (Litze) auf, der oder die von einem elektrisch isolierenden Mantel umhüllt ist oder sind. Die Mäntel der Leitungen und der Isolierkörper können aus gleichen oder aus unterschiedlichen Materialien, insbesondere unpolaren Kunststoffen bestehen. Es eignet sich u.a. jeder der oben genannten Kunststoffe.

Die Mäntel der Leitungen weisen vorzugsweise jeweils einen Kreisquerschnitt auf, in dessen Mitte der jeweilige Leiter angeordnet ist. Die Summe der Durchmesser der beiden Mäntel ist vorzugsweise kleiner als die Weite der Öffnung. Der zwischen den Leitungen angeordnete Isolierkörper weist eine solche Dicke auf, dass er gerade eben ohne oder mit geringer mechanischer Spannung zwischen die beiden Leitungen passt und diese dabei innen an die Öffnungswandung anlegt.

Vorzugsweise ist der Isolierkörper elastisch ausgebildet, sodass seine Dicke komprimierbar ist. Dazu kann er zwei voneinander weg und aufeinander zu federnde Schenkel aufweisen. Er kann aber auch im Ganzen verformbar als kompakter Körper ausgebildet sein. An seinen den Leitungen zugewandten Flächen kann er konkav ausgebildet sein, um die Leitungen örtlich festzulegen.

Bei einer Ausführungsform begrenzen die den Leitungen zugewandten Flächen maulartige Ausnehmungen, die über mehr als die Hälfte des Umfangs der darin angeordneten Leitung an dieser anliegen. Dadurch wird zwischen den beiden Leitern eine im Wesentlichen homogene Isolierung geschaffen, was Teilentladungen entgegenwirkt. Zusätzlich kann an den Flächen des Isolierkörpers, die an den Leitungen anliegen, eine leitende Schicht, z.B. eine Metallisierung angebracht sein. Die so geschaffene Äquipotentialfläche wirkt einer lokalen Konzentration des elektrischen Feldes und somit der Ausbildung von Teilentladungen entgegen. Es können auch an anderen Stellen leitfähige Bereiche vorgesehen sein. Z.B. kann in der Mittel- und Symmetrieebene zwischen den Leitungen eine leitende Schicht angeordnet sein. Damit lässt sich eine Symmetrierung des elektrischen Feldes bewirken.

Die Öffnung des Sensors kann kreisförmig ausgebildet sein. Zum Beispiel kann der Magnetkreis durch ein hohlzylindrisch oder als Torus ausgebildetes Teil gebildet sein. Andere Formen sind jedoch möglich, insbesondere können die Öffnung wie auch der Außenumriss des Sensors polygonal begrenzt sein. Es ist außerdem möglich, den Magnetkreis mit einer durch den Isolierkörper unterteilten Öffnung zu versehen. Der Isolierkörper kann mit dem Magnetkreis mechanisch verbunden sein. Z.B. kann er mit dem Magnetkreis durch eine Rastverbindung, Klemmverbindung, eine Klebeverbindung oder durch ein sonstiges geeignetes Mittel verbunden sein.

Bei einer bevorzugten Ausführungsform ist der Isolierkörper durch ein im Wesentlichen ebenes dreischenkliges Teil gebildet, bei denen zwei äußere Zungen dazu vorgesehen sind, den Sensor außen zu umgreifen. Von einem die beiden Zungen verbindenden Joch erstreckt sich eine innere Zunge parallel zu den äußeren Zungen so, dass sie in die Öffnung des Sensors einführbar ist. Dieser Isolierkörper kann zum Beispiel vor dem Durchfädeln der Leitungen durch den Sensor in die Öffnung des Magnetkreises eingesetzt werden, wobei er dann während und nach der Montage der Leitungen an dem Sensor verbleibt. Vorzugsweise sind die freien Enden der beiden äußeren Zungen mit nach innen weisenden Vorsprüngen (Nasen) versehen, mittels derer der Isolierkörper an dem Sensor verliersicher gehalten ist.

Weitere Einzelheiten vorteilhafter Ausführungsformen des Geräts sind Gegenstand von Unteransprüchen sowie der Zeichnung und der dazugehörigen Beschreibung. In der Zeichnung zeigen:
Figur 1 ein Gerät mit einem angeschlossenen monopolaren Instrument, das auf biologisches Gewebe einwirkt, welches über eine Neutralelektrode mit dem Gerät verbunden ist, in schematischer Darstellung,
Figur 2 das Gerät nach Figur 1 bei der Speisung eines bipolaren Instruments,
Figur 3 ein Stromsensor mit torusförmigen Magnetkreis, in perspektivischer Darstellung,
Figur 4 bis 8 den Stromsensor mit verschiedenen Isolierkörpern, jeweils in schematisierter Frontansicht,
Figur 9 den Stromsensor nach Figur 8, in Perspektivdarstellung und
Figur 10 den Isolierkörper des Stromsensors nach Figur 9, in schematisierter perspektivischer Darstellung.

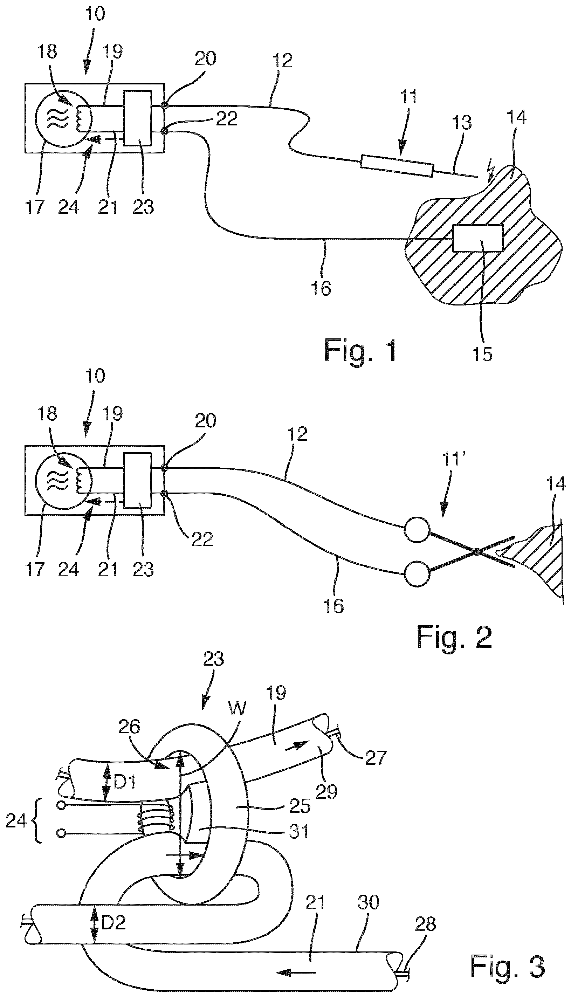

In Figur 1 ist ein Gerät 10 mit einem daran angeschlossenen Instrument 11 veranschaulicht, das mit dem gerät 10 über ein Kabel 12 verbunden ist. Das Instrument 11 weist eine Elektrode 13 zur Einwirkung auf biologisches Gewebe 14 auf, an dem eine Neutralelektrode 15 angebracht ist. Von dieser wird der von der Elektrode 13 in das Gewebe 14 eingebrachte Strom über ein Kabel 16 zu dem Gerät 10 rückgeführt.

Das Gerät 10 enthält einen Generator 17 zur Bereitstellung einer hochfrequenten Spannung bis zu bspw. 5 kVp, oder auch mehr, bei einer Frequenz zwischen 250 kHz und 2,5 MHz. Dieser Generator 17 weist einen Auskoppelkreis 18 auf, der über eine erste Leitung 19 mit einem ersten Geräteausgang 20 und über eine zweite Leitung 21 mit einem zweiten Geräteausgang 22 verbunden ist. Auch höhere Spannungen und/oder abweichende Frequenzen sind möglich.

Figur 2 veranschaulicht das gleiche Gerät 10 bei der Speisung eines bipolaren Instruments 11', beispielsweise beim Schnitt oder bei der Koagulation des Gewebes 14. Wiederum ist das Instrument 11' über zwei Kabel 12, 16 mit den Geräteausgängen 20, 22 verbunden, wobei die Kabel 12, 16 auch in Gestalt eines zwei- oder mehradrigen Kabels zusammengefasst sein können.

In dem Gerät 10 führen beide Leitungen 19, 21 durch einen Stromsensor 23, der zur Erfassung des zu dem Instrument 11 gelieferten Stroms und zur Erzeugung eines an dem Generator 17 gelieferten Messsignals 24 eingerichtet ist. Das Messsignal kann ein elektrisches oder anderweitiges (zum Beispiel optisches) Signal sein und zur Steuerung des Generators 17 herangezogen werden.

Zwischen den beiden Geräteausgängen 20, 22 und somit zwischen den Leitungen 19, 21 liegt eine den jeweiligen Behandlungsmode entsprechende Spannung zwischen wenigen 100 Vp und mehreren kVp (Kilovolt Spitzenspannung) an. Die beiden Leitungen 19, 21 sind dabei zusammen mit dem Stromsensor 23 in Figur 3 gesondert veranschaulicht. Dieser weist einen Magnetkreis 25 mit einer zentralen Öffnung 26 auf, durch die die beiden Leitungen 19, 21 hindurchgefädelt sind. Der Magnetkreis 25 kann beispielsweise ein torusförmiger oder hohlzylindrischer Körper, beispielsweise aus verlustarmem magnetischem Ferrit oder dergleichen, ausgebildet sein.

Der Magnetkreis 25 kann mit einer Sensorspule bewickelt sein, die an ihren Enden das Messsignal 24 bereitstellt. Der durch Leitungen 19, 21 fließende HF-Wechselstrom erzeugt in dem Magnetkreis ein magnetisches Wechselfeld, das in der Sensorspule das Messignal 24 z.B. als Spannungssignal induziert. Dem Messignal 24 können Störanteile überlagert sein, die von sporadischen Ladungsbewegungen in der Nachbarschaft der Messspule herrühren. Solche Ladungsverschiebungen können von Teilentladungen innerhalb elektrisch nichtleitender Elemente oder Objekte in der Nähe der Messspule herrühren.

Die beiden Leitungen 19, 21 sind beide so durch die Öffnung 26 gefädelt, dass der Strom, wie durch Pfeile angedeutet, gleichsinnig durch die Öffnung 26 fließt, um den zu dem Instrument 11, 11' gelieferten und von diesem zurückkommenden Strom aufsummierend zu messen. Die beiden Leitungen 19, 20 weisen dabei jeweils einen Leiter 27, 28 auf, der von einem isolierenden Mantel 29, 30 umgeben ist. Der jeweilige Leiter 27, 28 kann durch einen Draht oder auch durch ein Drahtbündel beispielsweise eine Litze gebildet sein. Der Mantel 29 weist vorzugsweise einen Kreisquerschnitt auf und besteht aus einem Kunststoff mit geringen dielektrischen Verlusten wie beispielsweise Polyethylen, Polytetrafluoräthylen oder anderen.

Die Isolationsstärke der Mäntel 29, 30 ist so bemessen, dass die Leiter 27, 28 auch bei den höchsten anzunehmenden Spannungen (zum Beispiel 5 kVp) durchschlagfest sind. Es zeigt sich, dass auch bei maximal dicken Mänteln 29, 30, bei denen die beiden Leitungen gerade eben noch durch die Öffnung 26 passen, in den Mänteln 29, 30 Ladungsverschiebungen auftreten können, die zwar hinsichtlich der Isolierung unbedenklich sind, dafür aber als Störströme in Erscheinung treten, die das Messignal 24 verfälschen. Dies gilt insbesondere bei der Erfassung von lediglich geringen Strömen in den Leitungen 19, 20, wie sie bei Arbeiten an hochohmigen (oder hochohmig gewordenem) Gewebe auftreten. Die Störsignal-Einstreuungen können das Messignal 24 schlimmstenfalls bis zur Unkenntlichkeit verfälschen, insbesondere, wenn das Messignal selbst Null sein sollte. Außerdem wären durch solche Teilentladungen eine Verzerrung der Kurvenform des Messsignals 24 und somit Fehlinterpretationen z.B. über den Gewebezustand möglich. Die Teilentladungen können dem Messignal ein Rauschsignal überlagern, dass zu Fehlmessungen führt.

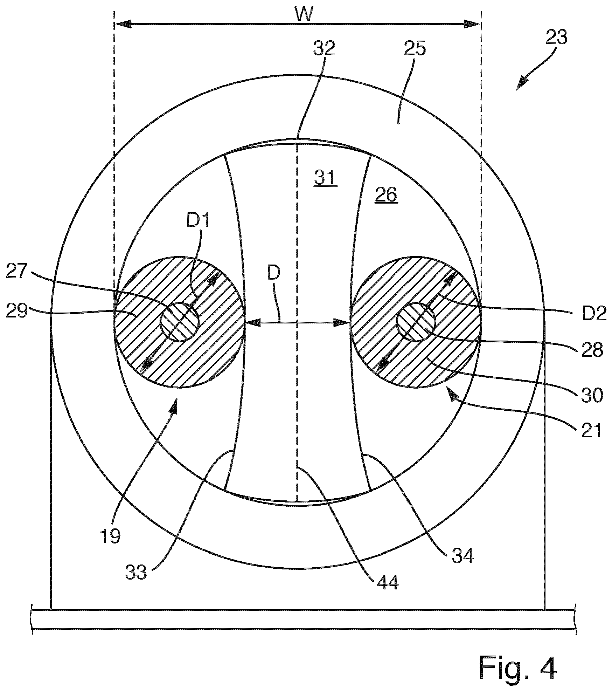

Bei dem erfindungsgemäßen Gerät 10 und Stromsensor 23 sind die Mäntel 29, 30 jedoch wesentlich dünner ausgebildet. Sie sind insbesondere so dünn, dass sie sich leicht und mit Spiel durch die Öffnung 26 fädeln lassen, die beispielsweise eine Weite von lediglich 5 mm aufweisen kann. Damit ist die Summe der Durchmesser D1 und D2 der beiden Leitungen 19, 21 geringer als die Weite W. Der Abstand zwischen den Leitungen 19, 21 innerhalb der Öffnung 26 wird dann durch einen Isolierkörper 31 maximiert, der zwischen den Leitungen 19, 21 angeordnet ist und diese voneinander weg gegen die Innenseite des Magnetkreises 25 und somit gegen die Wandung der Öffnung 26 drängt. Die Summe aus der Dicke D des Isolierkörpers und den Durchmessern D1 und D2 entspricht somit der Weite W, wie insbesondere auch aus Figur 4 hervorgeht.

Durch die Maximierung des Abstands der Leiter 27, 28 voneinander innerhalb der Öffnung 26 und die Anordnung des Isolierkörpers 31 zwischen den Leitungen 19, 21 werden Teilentladungen in den Mänteln 29, 30 und somit im Stromsensor 23 vermieden. Es handelt sich um Teilentladungen, welche die Detektion des Stroms in den Leitungen 19, 20 stören und damit Funken oder tatsächlich nicht vorhandene Gewebereaktionen oder -Eigenschaften vortäuschen könnten. Durch die Unterdrückung von Teilentladungen innerhalb des Stromsensors 23 und der damit einhergehenden geringeren Störung des Messsignals 24 wird eine verbesserte Analyse der erfassten Ströme einschließlich ihrer zeitlichen Verläufe möglich, sodass verbesserte Rückschlüsse auf die Arbeit des Instruments 11, 11' sowie den Zustand des Gewebes 14 sowie dessen Veränderung gezogen werden können.

Die vorstehenden Ausführungen gelten uneingeschränkt für die nachfolgende Beschreibung abweichender Ausführungsformen der Erfindung:

Zunächst wird darauf hingewiesen, dass der Stromsensor 23 nach Figur 3 als Summenstromsensor beschaltet ist. Er kann jedoch auch als Differenzstromsensor ausgebildet sein, indem eine der Leitungen 19 oder 21 entgegen dem in Figur 3 dargestellten Richtungssinn durch die Öffnung 26 geführt ist. Auch hier liegt die volle Spannungsdifferenz zwischen den beiden Leitungen 19, 21 an, wobei allerdings von dem Magnetkreis 25 lediglich noch die durch z.B. kapazitive Leckströme verursachten kleine Differenz zwischen den beiden Strömen in den Leitungen 19, 21 erfasst wird. Gerade dabei ist aber die Vermeidung von Teilentladungen und somit die Vermeidung von Störungen des Signals besonders nützlich.

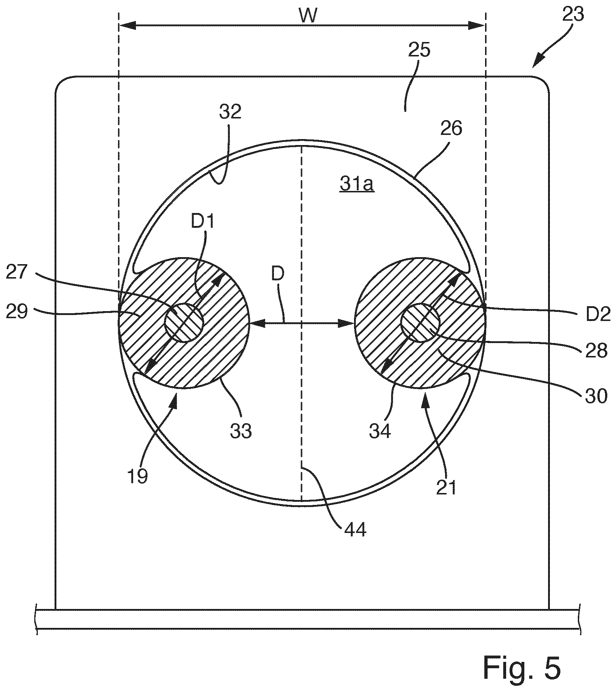

Der Magnetkreis 25 kann außer der in Figur 3 dargestellten Torusform auch hohlzylindrisch sein, wie es Figur 4 veranschaulicht. Außerdem kann er innen und/oder au-ßen eine polygonale Form aufweisen, wie die Figuren 5 bis 9 veranschaulichen. Die in den Figuren 5 bis 9 dargestellten verschiedenen Isolierkörper 31a, 31b, 31c, 31d können aber auch bei den Magnetkreisen 25 nach Figur 3 oder 4 Anwendung finden.

Der Isolierkörper 31a nach Figur 5 weist an seinem Außenumfang eine Außenfläche 32 auf, die der Form der Wandung der Öffnung 26 angepasst ist und beispielsweise einem Zylinder folgt. Die den Leitungen 19, 21 zugewandten Flächen 33, 34 sind sowohl bei dem Isolierkörper 31 nach Figur 4 als auch bei dem Isolierkörper 31a nach Figur 5 konkav ausgebildet. Bei der Ausführungsform nach Figur 5 umgreifen die Flächen 33, 34 die Leitungen 19, 21 und liegen an diesen an. Beide Isolierkörper 31, 31a können starr, zum Beispiel aus einem mineralischen Material wie Keramik, oder alternativ aus einem Kunststoff ausgebildet sein. Im letzten Fall können sie etwas kompressibel ausgebildet sein, um einen festen Sitz in der Öffnung 26 zu sichern.

Es ist möglich, die Flächen 33, 34 mit einer elektrisch leitfähigen Schicht zu versehen. Diese bildet eine Äquipotentialfläche, die eine lokale Konzentration des elektrischen Feldes verhindert oder zumindest stark reduziert. Alternativ oder zusätzlich kann in der symmetrisch zwischen den beiden Leitern 27, 28 liegenden Mittelebene eine elektrisch leitende Schicht 44 vorgesehen sein. Auch diese trägt zur Homogenisierung des elektrischen Feldes bei.

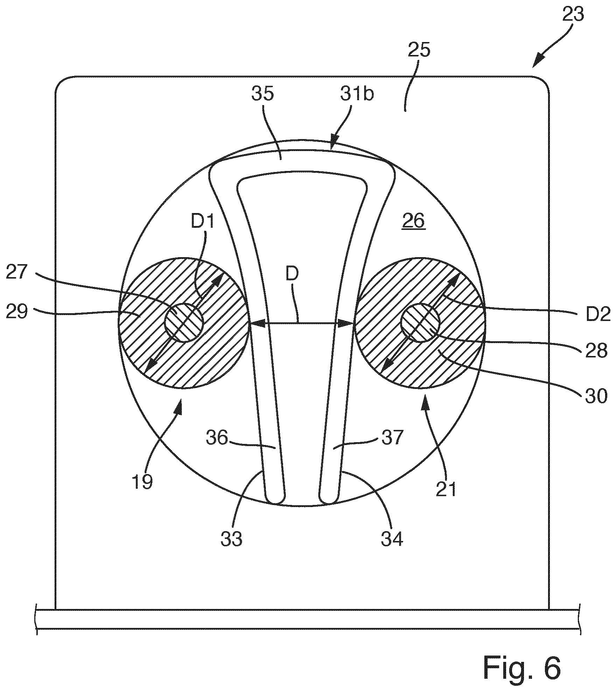

Eine abgewandelte Ausführungsform des Isolierkörpers zeigt Figur 6 in Gestalt des Isolierkörpers 31b. Der Isolierkörper 31b weist zwei von einem gemeinsamen Rückenabschnitt 35 sich weg erstreckende Schenkel 36, 37 auf, die aus Isoliermaterial ausgebildet sind und die Leitungen 19, 21 voneinander wegdrängen. Die Schenkel 36, 37 sind somit federnd angeordnet und ausgebildet. Der Isolierkörper 31b kann durch ein U-förmiges Kunststoffprofil gebildet sein. Die Schenkel 36, 37 des Isolierkörpers 31b sind nur schwach gekrümmt. Alternativ könne sie auch an die Leitungen 19, 20 außen anschmiegend ausgebildet sein. In diesem Fall kann es zweckmäßig sein, an den Flächen 33, 34 eine Metallisierung anzubringen.

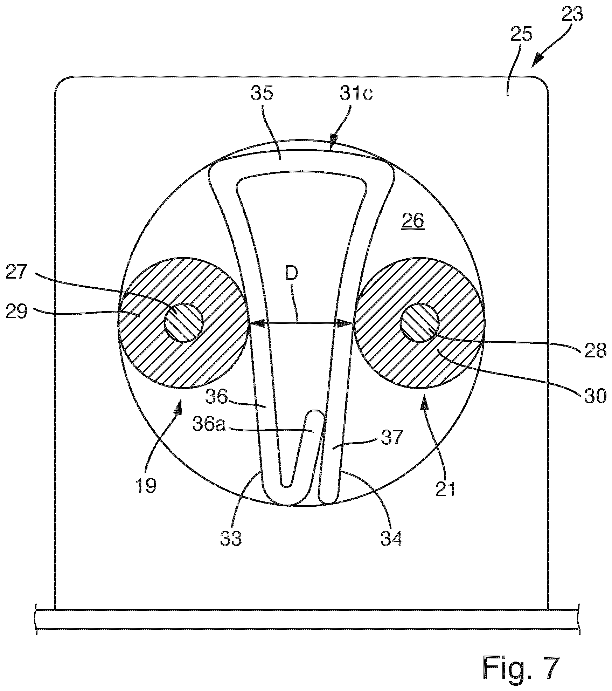

Dabei ist es auch möglich, dass die Schenkel 36, 37 nicht nur über den Rückenabschnitt 35 miteinander verbunden sind, wie es Figur 6 veranschaulicht. Vielmehr kann der Isolierkörper 31c nach Figur 7 ausgebildet sein. Bei diesem wird durch einen einwärts abgewinkelten Abschnitt 36a eines der Schenkel 36, 37, der sich an dem jeweils anderen Schenkel (hier Schenkel 37) abstützt, eine zusätzliche Federspannung aufgebaut.

Alle Isolierkörper 31, 31a, 31b, 31c können die gleiche axiale Länge (in den Figuren 4 bis 7 senkrecht zur Zeichenebene) haben, wie die Öffnung 26. Vorzugsweise sind sie jedoch etwas länger ausgebildet, um Teilentladungen zwischen den Leitungen 19, 21 nicht nur in der Öffnung 26, sondern auch in deren Nähe zu unterbinden.

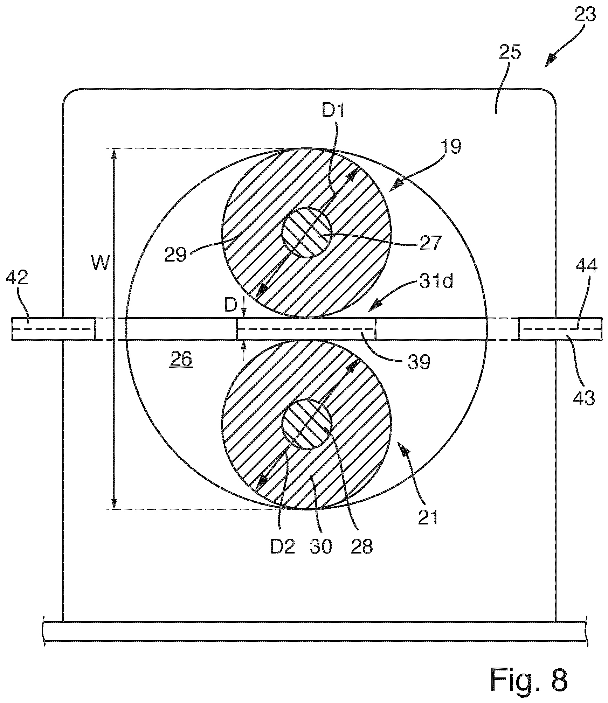

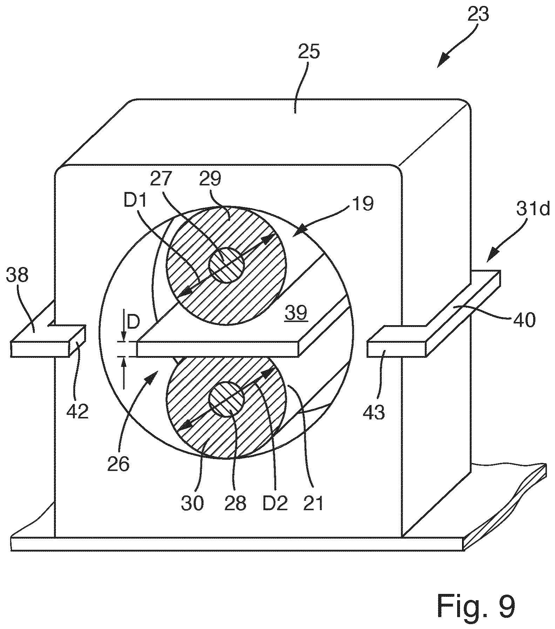

Eine weitere besonders handhabungsfreundliche und auch wirksame Isolierkörpervariante ist der Isolierkörper 31d, nach Figur 8 bis 10. Er ist vorzugsweise aus flexiblem Flachmaterial, zum Beispiel einem flachen flexiblen Kunststoffmaterial ausgeschnitten und weist drei in einer gemeinsamen Ebene liegende streifenförmige Zungen 38, 39, 40 auf, die sich parallel zueinander von einem Rückenabschnitt 41 weg erstrecken. Die äußeren beiden Zungen 38, 40 können an ihren freien Enden nach innen gerichtete Abschnitte 42, 43 aufweisen, die dazu geeignet sind, den Magnetkreis 25, wie es Figur 9 zeigt, zu übergreifen, während die mittlere Zunge 39 zwischen den Leitungen 19, 21 liegt.

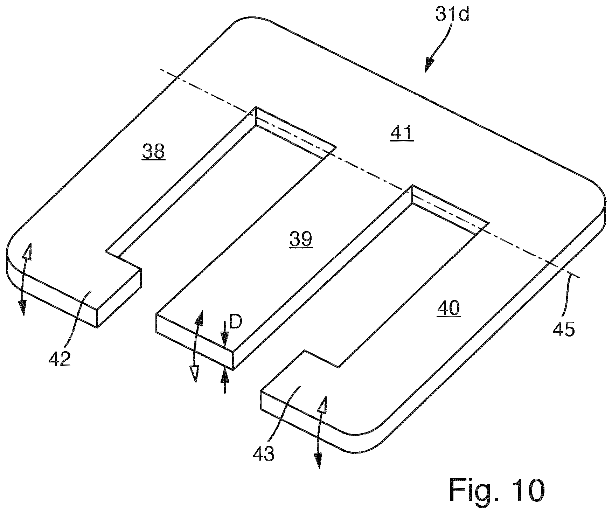

Die mittlere Zunge 39 und vorzugsweise auch die äußeren Zungen 38, 40 sind dabei an dem Rückenabschnitt 41 vorzugsweise flexibel gehalten. Die mittlere Zunge 39 lässt sich aus der von den Zungen 38, 40 definierten Ebene herausbewegen. Die äußeren Zungen 38, 40 lassen sich in Gegenrichtung auslenken, wie in Figur 10 durch (weiße und schwarze) Pfeile angedeutet ist. Die Zungen 38, 39, 40 können dabei um eine in dem Rückenabschnitt 41 oder in dessen Nähe liegende Schwenkachse 45 schwenken, wobei sie sich auch selbst flexibel krümmen können. Die Schwenkachse 45 markiert das Zentrum eines Verwindungsbereichs des insoweit flexiblen Isolierkörpers 31d.

Der Isolierkörper 31d kann vor der Montage der Leitungen 19, 21 mit dem Magnetkreis 25 verbunden werden, indem die Zungen 38, 39, 40 durch gegensinniges Verwinden soweit gespreizt werden, dass sich die mittlere Zunge 39 in die Öffnung 26 einführen lässt. Nach Freigabe schwenken alle Zungen 38, 39, 40 in eine Ebene zurück, wobei die äu-ßeren Zungen 38, 40 den Magnetkreis 25 umgreifen. Die mittlere Zunge 39 kann dabei eine mit der Weite der Öffnung 26 übereinstimmende Breite aufweisen oder auch etwas schmaler ausgebildet sein. Jedenfalls separiert sie die beiden Leitungen 19, 21. Die Zunge 39 weist eine Dicke D auf, die in Summe mit den Durchmessern D1, D2 der Leitungen 19, 21 der Weite W der Öffnung 26 entspricht.

Für jeden der Isolierkörper 31, 31a bis 31d gilt, dass dieser eine oder mehrere leitende Schichten 44 aufweisen kann. Bei den Isolierkörpern 31, 31a, 31d sind diese Schichten vorzugsweise in einer Mittelebene zwischen den Leitungen 19, 21 angeordnet und definieren somit eine Äquipotentialebene. Diese kann dazu beitragen, lokale Feldinhomogenitäten zu vermeiden und somit auch Teilentladungen zu reduzieren. Es ist auch möglich, die Flächen 33, 34 jeweils mit einer leitenden Schicht zu versehen.

Erfindungsgemäß wird für einen Stromsensor 23 eines Geräts 10 zur Speisung eines Instruments 11, 11' ein ringförmiger Magnetkreis 25 vorgesehen, durch den zwei das Instrument 11 und eine Neutralelektrode 15 oder das Instrument 11' speisende Leitungen 19, 21 geführt sind. Die Mäntel 29, 30 der beiden Leitungen 19, 21 weisen eine in ihrer Durchschlagfestigkeit auf die Spannungsbelastung der Leitungen 19, 21 bemessene hinreichende Dicke auf. Diese ist jedoch so weit begrenzt, dass zwischen die beiden Leitungen 19, 21 noch ein Isolierkörper 31 passt, der wie die Mäntel 28, 30 aus einem unpolaren hochisolationsfähigen Isolatormaterial besteht, beispielsweise einem Kunststoff mit geringem Verlustwinkel. Der Isolierkörper 31, 31a bis 31d, reduziert Teilentladungen im Bereich des Stromsensors 23, die ansonsten zu elektromagnetischen Emissionen und somit zur Störung des von dem Stromsensor 23 erzeugten Signals 24 führen könnten.

### Bezugszeichen:

- 10: Gerät
- 11: Instrument
- 12: Kabel
- 13: Elektrode
- 14: Gewebe
- 15: Neutralelektrode
- 16: Kabel
- 17: Generator
- 18: Auskoppelkreis
- 19: erste Leitung
- 20: erster Geräteausgang
- 21: zweite Leitung
- 22: zweiter Geräteausgang
- 23: Stromsensor
- 24: Messsignal
- 25: Magnetkreis
- 26: Öffnung
- 27: erster Leiter
- 28: zweiter Leiter
- 29: erster Mantel
- 30: zweiter Mantel
- 31: Isolierkörper
- 32: Außenfläche
- 33, 34: Flächen
- 35: Rückenabschnitt
- 36, 37: Schenkel des Isolierkörpers 31b oder 31c
- 36a: Vorsprung
- 38 - 40: Schenkel des Isolierkörpers 31d
- 41: Rückenabschnitt des Isolierkörpers 31d
- 42, 43: Vorsprünge
- 44: elektrisch leitende Schicht
- 45: Schwenkachse

## Patentansprüche

1. Gerät (10) zur Speisung eines elektrochirurgischen Instruments (11, 11') mit einem Behandlungsstrom, mit einem Generator (17), der einen Auskoppelkreis (18) aufweist, der über eine erste Leitung (19) mit einem ersten Geräteausgang (20) zur Speisung des Instruments (11, 11') und über eine zweite Leitung (16) mit einem zweiten Geräteausgang (22) zur Rückführung des Stroms verbunden ist,
mit einem Stromsensor (23) insbesondere zur Erfassung der Summe oder Differenz eines in der ersten Leitung (19) fließenden Stroms und eines in der zweiten Leitung (21) fließenden Stroms,
wobei der Stromsensor (23) einen Magnetkreis (25) mit einer zentralen Öffnung (26) aufweist, durch die sowohl die erste Leitung (19) als auch die zweite Leitung (21) geführt ist,
**dadurch gekennzeichnet,**
**dass** in der zentralen Öffnung (26) zwischen den beiden Leitungen (19, 21) ein Isolierkörper (31, 31a - 31d) angeordnet ist.

2. Gerät nach Anspruch 1, **dadurch gekennzeichnet, dass** die Leitungen (19, 21) jeweils einen zentralen elektrischen Leiter (27, 28) und einen diesen umhüllenden elektrisch isolierenden Mantel (29, 30) aufweisen.

3. Gerät nach Anspruch 2, **dadurch gekennzeichnet, dass** der Mantel (29, 30) jeweils einen Kreisquerschnitt aufweist, in dessen Mitte der jeweilige Leiter (27, 28) angeordnet ist.

4. Gerät nach Anspruch 2 oder 3, **dadurch gekennzeichnet, dass** jeder Mantel (29, 30) einen Durchmesser (D1, D2) und die Öffnung (26) eine Weite (W) aufweist, wobei die Summe der Durchmesser (D1, D2) geringer ist, als die Weite (W).

5. Gerät nach Anspruch 4, **dadurch gekennzeichnet, dass** der Isolierkörper (31, 31a-d) eine Dicke (D) aufweist, wobei die Summe aus den Durchmessern (D1, D2) und der Dicke (D) mit der Weite (W) übereinstimmt.

6. Gerät nach Anspruch 4, **dadurch gekennzeichnet, dass** der Isolierkörper (31, 31a-d) eine Dicke (D) aufweist, wobei die Summe aus den Durchmessern (D1, D2) und der Dicke (D) größer ist, als die Weite (W).

7. Gerät nach einem der vorstehenden Ansprüche, **dadurch gekennzeichnet, dass** der Isolierkörper (31, 31a-d) elastisch ausgebildet ist.

8. Gerät nach einem der vorstehenden Ansprüche, **dadurch gekennzeichnet, dass** der Isolierkörper (31, 31a-c) in seiner Dicke (D) komprimierbar ist.

9. Gerät nach einem der vorstehenden Ansprüche, **dadurch gekennzeichnet, dass** der Isolierkörper (31b, 31c) zwei voneinander weg und aufeinander zu federnde Schenkel (36, 37) aufweist.

10. Gerät nach einem der vorstehenden Ansprüche, **dadurch gekennzeichnet, dass** der Isolierkörper (31, 31a) an den Leitungen (19, 21) zugewandten Flächen (33, 34) konkav gewölbt ausgebildet ist.

11. Gerät nach einem der vorstehenden Ansprüche, **dadurch gekennzeichnet, dass** die Öffnung (26) kreisförmig ausgebildet ist.

12. Gerät nach einem der vorstehenden Ansprüche, **dadurch gekennzeichnet, dass** der Isolierkörper (31a) eine an die Öffnung (26) angepasste Außenform mit zwei an die Leitungen (19, 20) angepassten Flächen (33, 34) aufweist, die jeweils eine maulartige Ausnehmung begrenzen.

13. Gerät nach Anspruch 1, **dadurch gekennzeichnet, dass** der Isolierkörper (31d) einen den Magnetkreis (25) umgreifenden Halteabschnitt (41; 38, 42; 40, 43) aufweist.

14. Gerät nach Anspruch 13, **dadurch gekennzeichnet, dass** der Isolierkörper (31d) eine sich von einem zu dem Halteabschnitt (41; 38, 42; 40, 43) gehörigen Rückenabschnitt (41) weg durch die Öffnung (26) erstreckende Zunge (39) aufweist.

15. Gerät nach Anspruch 14, **dadurch gekennzeichnet, dass** die Zunge (39) und der Halteabschnitt (41; 38, 42; 40, 43) als einstückiges flaches Teil ausgebildet sind, wobei die Zunge (39) und der Halteabschnitt (41; 38, 42; 40, 43) gegeneinander schwenkbar sind.
